# FASCICULE DE BREVET EUROPEEN

(11) **EP 0 375 578 B1**
(45) Date de publication et mention de la délivrance du brevet: **07.09.1994**
(21) Numéro de dépôt: 89430036.7
(22) Date de dépôt: 20.12.1989
(51) Int. Cl.: A61C 3/02, B23K 26/00

(54) **Installation utilisant l'effet laser, pour la coupe ou la vaporisation de matériaux et tissus divers**
Vorrichtung zum Schneiden oder Verdampfen von Material und verschiedenen Stoffen unter Anwendung der Laserwirkung
Device for the cutting or vaporizing of material and different substances by using the laser effect

(30) Priorité: 21.12.1988 FR 8817549
(43) Date de publication de la demande: 27.06.1990
(73) Titulaire: LASER MEDICAL TECHNOLOGY, Inc., San Clemente, California 92673 (US)
(72) Inventeur: Levy, Guy, F-13004 Marseille (FR)
(74) Mandataire: Marek, Pierre, Cabinet Marek

(56) Documents cités:
- WO-A-86/06269
- DE-A- 2 828 322
- DE-A- 3 637 568
- US-A- 4 249 060
- US-A- 4 398 790
- JOURNAL OF APPLIED PHYSICS vol. 59, no. 6, mars 1986, pages 1829-1833, Woodbury, New York, US; A.D. McLACHLAN: "Improved coupling coefficient of laser radiation to aluminum by means of absorptive polymer coatings"

## Description

La présente invention concerne une installation pour la coupe ou la vaporisation de matériaux et de tissus divers, tels que peau, émail, bois, etc. Selon un exemple d'application avantageuse, mais non limitative, l'installation selon l'invention permet le traitement de la carie dentaire et la découpe de la dentine. Dans cette application, l'objet de l'invention permet notamment l'identification de la carie, l'élimination ou le curetage des tissus pathologiques, puis la découpe de la dentine et de l'émail avec une grande précision à l'aide d'un faisceau laser. Dans les applications de ce genre, le recours au laser parait devoir s'avérer très intéressant car, notamment grâce à l'emploi de fibres optiques, le rayonnement laser peut être concentré sur une surface très réduite, de sorte qu'il est donc compatible avec les contraintes dimensionnelles des procédures dentaires. En outre, les procédures au laser peuvent être réalisées sans recourir à un anesthésique.

Si un certain nombre d'appareils de ce type ont été proposés, leur utilité pratique n'est pas établie, en particulier en raison du fait que même les plus performants des dispositifs déjà proposés ne sont utiles que dans des conditions limitées et définies de façon très précise.

Les problèmes rencontrés dans la technique connue actuellement quant à l'utilisation des lasers en odontologie, concernent principalement :
- 1 ) la longueur d'onde et l'absorption de l'énergie par les tissus concernés ;
- 2 ) l'effet, sur l'organe dentaire ou sur les tissus environnants, des différents paramètres tels que :
   - a) intensité ;
   - b) durée de l'impulsion ;
   - c) taux de répétition.

On connait par exemple (US-A-3.821.510), un procédé et un appareil de coupe de matériaux divers incluant l'émail dentaire, au moyen d'un rayon laser produit par un laser au neodyme ou autre appliqué sur le matériau en même temps qu'un fluide gazeux de refroidissement.

Toutefois, le procédé et l'appareil décrits dans ce document ne permettent pas la coupe de tissus dentaires tels que l'émail et la dentine sans provoquer d'effets secondaires nuisibles sur les tissus environnants, en raison de l'utilisation d'un rayonnement laser dont les caractéristiques décrites ne permettent pas une coupe nette et précise des tissus, et l'utilisation d'un fluide gazeux de refroidissement dont l'effet est insuffisant pour s'opposer efficacement à la diffusion de la chaleur dans la région environnant la zone d'application du rayonnement.

On a constaté, en effet, que si une longueur d'onde particulière peut avoir un effet de coupe intrinsèque sur l'émail ou la dentine, l'utilisation pratique du rayonnement sur cette longueur d'onde pour les procédures dentaires dépend dans une large mesure de la forme sous laquelle la radiation est utilisée, en ce qui concerne la quantité d'énergie, la durée et la fréquence d'impulsion. On a constaté spécifiquement que les tentatives d'application de cette radiation sous forme d'impulsions à énergie élevée et de courte durée, produisent une élévation de température très localisée, qui se traduit par une dilatation thermique différentielle de nature à provoquer des dommages mécaniques à la dent ainsi que des lésions vasculaires au tissu gingival. Inversement, les impulsions à faible énergie et de longue durée entrainent un échauffement plus généralisé de la dent, qui provoque l'inconfort du patient ainsi que des dommages à la pulpe par la chaleur.

La dent et son environnement sont composés de tissus durs (l'émail, la dentine) et de la muqueuse qui entoure cette dent.

L'émail et la dentine sont composés :
- d'un élément minéral, l'hydroxyapatite qui se présente à l'état amorphe dans la dentine et sous forme cristalline dans l'émail ;
- d'éléments ou tissus organiques et d'eau.

Les longueurs d'onde utilisables sont directement influencées par les composants des différents tissus qui réagissent différemment au rayonnement laser.

L'hydroxyapatite :
- absorbe les rayonnements laser sur les longueurs d'onde comprises entre 9 µm et 11 µm, produites par les lasers à CO₂ ;
- absorbe égalemant les rayonnements laser sur les longueurs d'onde comprises entre 0.50 µm et 1.06 µm, susceptibles d'être produites par les lasers YAG.

L'émail à l'état cristallin, de par ses qualités optiques et son pouvoir de réflexion, absorbe beaucoup moins bien ces différentes longueurs d'onde.

La dentine comporte un pourcentage organique de l'ordre de 40 % à haut pourcentage d'eau ; ce pourcentage organique augmentant considérablement lors de l'atteinte carieuse.

L'absorption du tissu dentinaire aux différentes longueurs d'onde est donc influencée par l'absorption de l'énergie émise, par sa composante aqueuse. Plus l'absorption d'énergie par l'eau est importante, moindre est l'énergie disponible pour absorption par les autres composantes.

La longueur d'onde des lasers à CO₂, largement absorbée par l'eau, est pratiquement inactive sur les tissus minéraux.

La longueur d'onde 1.06 µm des lasers à YAG-Neodyme ou ND YAG, peu absorbée par l'eau, est donc plus active sur le tissu minéral, et, par conséquent, son effet de coupe est plus important sur les tissus minéralisés.

La longueur d'onde 0.532 µm, transparente dans l'eau, n'est pas absorbée par celle-ci, de sorte que le rayonnement ne peut être efficace sur les tissus minéralisés que si une énergie suffisamment élevée, et donc dangereuse, est mise en oeuvre.

Les tissus organiques ou tissus vasculaires sont influencés essentiellement par l'absorption aux différentes longueurs par l'eau et l'hémoglobine.

Le rayonnement sur les longueurs d'onde émises par les lasers à CO₂ coupe les tissus organiques, en raison de son absorption par l'eau.

Le rayonnement sur la longueur d'onde 1.06 µm des lasers YAG n'a aucun effet de coupe sur les tissus organiques avec des intensités relativement importantes, car non absorbée par l'eau.

La longueur d'onde 0.532 µm, bien absorbée par l'hémoglobine, coupe les tissus vascularisés ou tissus tendres.

D'autre part, l'absorption, aux différentes longueurs d'onde, est considérablement influencée par la couleur. Ainsi :
- la longueur d'onde du laser à CO₂ est transparente au noir ;
- la longueur d'onde du laser ND YAG est très largement absorbée par le noir.

Les lasers à CO₂ utilisés en art dentaire ont des indications limitées car leur longueur d'onde est bien absorbée par l'émail et la dentine, mais son action est toutefois déviée par la compétition due à l'eau. Les intensités élevées qui seraient nécessaires à la coupe de ces tissus seraient très dangereuses car susceptibles d'intéresser les tissus organiques.

Les lasers YAG ne sont toutefois pas encore proposés pour l'art dentaire, car ils ne peuvent être actifs que dans des conditions bien précises.

Est connue, en médecine, l'utilisation de lasers YAG travaillant en continu, donc non pulsés ; toutefois leur emploi pour des interventions sur l'émail et la dentine sont décevants.

Est également connu, l'emploi de lasers pulsés travaillant à la nano-seconde et apportant une énergie plus ou moins élevée à des taux de répétition plus ou moins rapides. L'avantage est représenté par le fait qu'il est possible de réguler la cadence de tir ainsi que l'énergie apportée à chaque tir, en particulier en fonction de la conduction thermique des tissus minéraux.

Les indications de ces types de lasers sont cependant limitées, car :
- les lasers pulsés avec une énergie élevée créent une onde de choc préjudidiable à la pulpe vivante et entraînent des phénomènes de séparation de la jonction émail/dentine, ainsi que des craquelures qui sont aussi la conséquence de la variation thermique apportant dilatation et rétraction près de la zone de tir.

On connait enfin divers lasers YAG travaillant à la milli-seconde (100 à 200), mais ils ne peuvent être utilisés en odontologie car la diffusion thermique étant plus importante, il convient d'avoir un taux de répétition peu élevé (2 Hz), ce qui rendrait le laser peu actif sur la dent si l'on veut maintenir une température supportable.

L'invention vise à remédier aux inconvénients de la situation exposée ci-dessus en régulant l'énergie de coupe et par conséquent, l'effet thermique, par la prévision de paramètres spécifiques pour le rayonnement laser et par la mise en oeuvre de moyens d'application n'influençant pas l'efficacité élective du rayon laser.

Selon le dispositif de l'invention, on utilise pour le traitement de la carie dentaire et la coupe de la dentine, un laser spécifique de type YAG présentant notamment les caractéristiques suivantes :
- longueur d'onde de l'ordre de 1,06 µm ou de l'ordre de 0,532 µm ;
- émission d'une énergie pulsée comprise entre 0 et 250 mJ et, de préférence, entre 10 et 50 millijoules (mJ) ;
- cadence ou fréquence de tir de l'ordre de 50 Hz ;
- durées des pulsations ou impulsions comprises entre 100 et 300 micro-secondes.

L'échauffement occasionné par l'apport d'énergie sur les tissus dentaires est contrôlée par un fluide de refroidissement tel qu'un mélange air/eau projeté sur la zone de tir, l'eau transparente à la longueur d'onde n'affectant pas l'efficacité de coupe du laser sur les tissus durs.

Enfin, on peut réduire l'énergie apportée en induisant la formation d'un plasma au-dessus de la zone de coupe, en apportant une coloration noire qui absorbera totalement l'énergie laser.

Selon une autre caractéristique, l'installation selon l'invention comprend une pièce à main utilisant le rayon laser et qui peut être avantageusement réalisée sous une forme rappelant celle des contre-angles classiques couramment utilisés en art dentaire, cet appareil comprenant un corps creux de forme allongée dont l'une des extrémités constitue, ou est agencée pour constituer une tête dans laquelle est installé un dispositif de focalisation auquel aboutit l'extrémité d'une fibre optique logée dans ledit corps et permettant de transporter le rayon laser jusqu'audit dispositif de focalisation, ladite tête étant agencée pour recevoir un instrument d'application interchangeable complétant la pièce à main et constitué par une fibre optique souple dont une partie s'étend hors de ladite tête ; le dispositif de focalisation étant orienté de manière à diriger le rayon laser focalisé sur l'extrémité interne de ladite fibre optique interchangeable, lorsque cette dernière est en place.

La mise en oeuvre de l'installation selon l'invention a notamment pour avantages de permettre une utilisation effective des lasers de type YAG pour effectuer la coupe ou la volatilisation de matériaux et tissus divers, (par exemple et de manière avantageuse, la détection et la destruction de la carie et la coupe de la dentine saine), notamment dans des cavités canalaires telles que canaux dentaires.

Les buts, caractéristiques et avantages ci-dessus, et d'autres encore, ressortiront mieux de la description qui suit et des dessins annexés dans lesquels :
La figure 1 est une vue en coupe longitudinale et à caractère schématique d'une installation selon l'invention comprenant un premier mode d'exécution d'une pièce à main permettant l'application de l'énergie focalisée sur les surfaces d'intervention, et la projection d'un mélange air/eau de refroidissement.
La figure 2 est une vue en coupe longitudinale et à caractère schématique d'un deuxième mode d'exécution d'une telle pièce à main.

On se réfère auxdits dessins pour décrire des exemples avantageux, bien que nullement limitatifs, de mise en oeuvre et de réalisation de l'installation selon l'invention.

Cette installation est notamment remarquable par le fait que la volatilisation ou la coupe des matériaux telles que la détection et la destruction de la carie, et la coupe de la dentine saine, sont opérées au moyen d'un laser spécifique de type YAG comportant les caractéristiques originales ci-après :
- une longueur d'onde de 1,060 µm ou de 0,532 µm ;
- une capacité d'émission d'énergie comprise entre 0 et 250 millijoules (mJ) et, de préférence, entre 10 et 50 millijoules (mJ) par pulsation ou impulsion ;
- une capacité de cadence ou de fréquence de tir de 50 Hz ;
- des durées de pulsations ou impulsions comprises entre 100 et 300 micro-secondes.

La durée d'impulsion de 100 à 300 microsecondes est suffisamment longue pour éviter de soumettre le tissu à traiter à des chocs thermiques, mais suffisamment courte pour permettre de maitriser efficacement l'effet d'échauffement.

Pour la mise en oeuvre de l'invention, le rayon laser doit être focalisé ou concentré sur un point de l'ordre de 200 à 600 microns sur le site d'intervention, ce qui permet le transport de l'énergie à l'intérieur de canaux de faibles diamètres (par exemple : canaux dentaires), à l'aide de fibres optiques présentant ce diamètre.

D'autre part, la diffusion thermique est avantageusement limitée grâce à l'apport d'un fluide de refroidissement tel que, par exemple, un mélange eau/air qui s'effectue simultanément aux tirs et dont les débits sont réglés pour être proportionnels au coefficient d'absorption de l'eau qui :
- pour une longueur d'onde de 1,06 µm, est de 0,500, et qui
- pour une longueur d'onde de 0,532 µm, est de 0,003.

L'émail déminéralisé opaque, ainsi que la dentine pathologique, sont volatilisés au point focal ou légèrement après le point focal, par la formation d'un plasma qui a tendance à augmenter au point de coupe, ce qui permet de réduire l'énergie utilisée entre 10 et 20 millijoules.

Les expériences faites dans la destruction de la carie, ont démontré que l'absorption plus marquée de la dentine pathologique était en partie due à un changement de teinte découlant du processus carieux.

Pour obtenir la coupe de la dentine saine, on a prévu, pour la mise en oeuvre de l'invention, de noircir la zone de coupe, par exemple, de manière avantageuse, à l'aide d'une pointe de graphite noir mélangé à une colle qui évite l'élimination hâtive du produit de noircissement, par l'action du mélange air/eau du refroidissement opéré en cours de coupe.

Dans ce cas, les caractéristiques de tir sont identiques à celles décrites précédemment pour la destruction de la dentine pathologique.

Pour éviter un échauffement excessif au cours de la coupe, on associe avantageusement un mélange air/eau projeté sur la zone d'intervention à des pressions : air : 2 bars environ/ eaux : 1 litre/heure. Dans ce cas, l'échauffement est compatible avec les fonctions vitales d'une pulpe dentaire.

Le laser mis en oeuvre dans l'installation selon l'invention, peut être un laser exclusivement réservé à la coupe des tissus durs. Sa longueur d'onde est alors de 1,06 µm. A cette longueur d'onde et aux intensités susmentionnées, l'onde n'a aucun effet de coupe sur les muqueuses.

Il est possible d'utiliser un doubleur de fréquence, au moyen d'un montage optique très simple permettant de doubler la longueur d'onde sur un laser YAG ayant une longueur d'onde de 1,06 µm, pour la porter à 0,532, un tel montage étant uniquement mis en oeuvre pour la chirurgie muqueuse, car la longueur d'onde à 0,532 µm est bien absorbée par l'hémoglobine.

L'invention concerne également une installation comportant une pièce à main permettant de conduire le rayon laser et d'appliquer le rayon focalisé à un point d'impact, sur une paroi dentaire ou dentinaire, par exemple à l'intérieur d'un canal radiculaire.

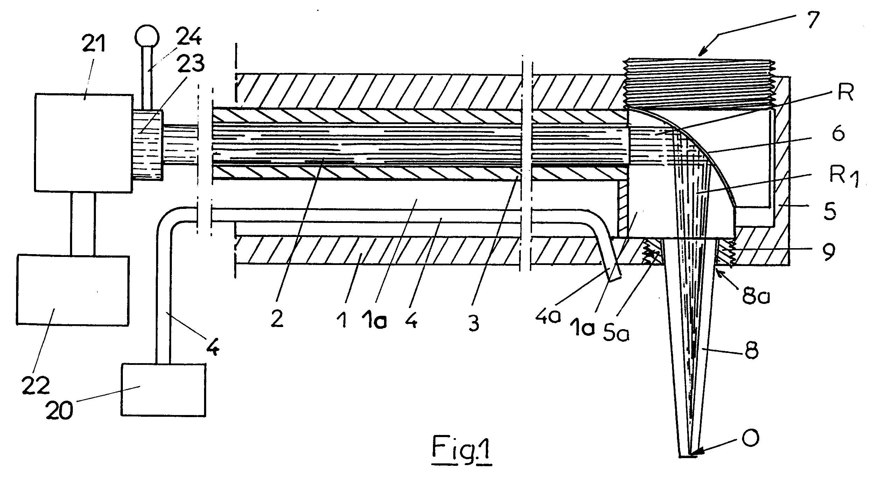

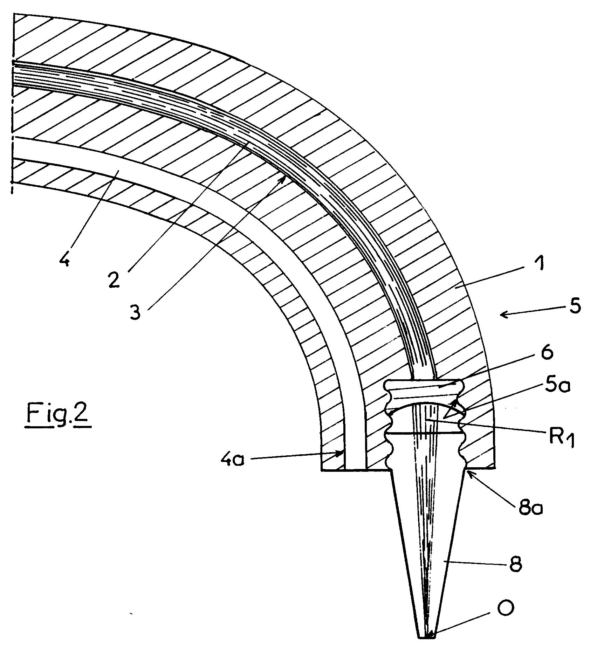

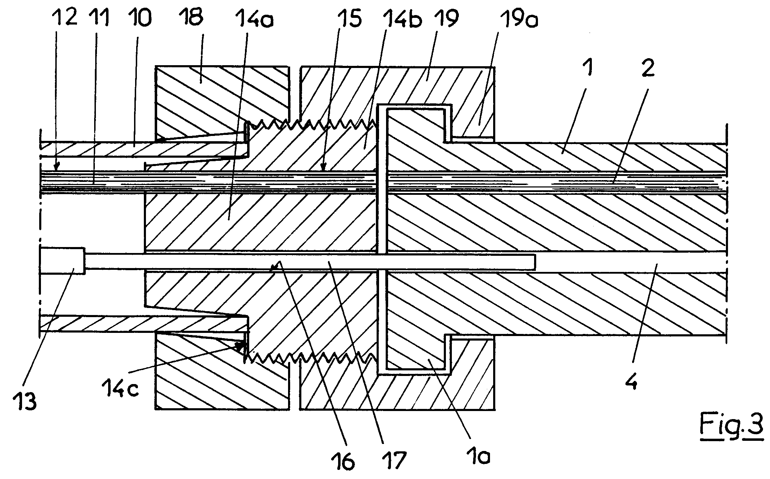

Cette pièce à main comprend un corps creux 1 de forme allongée qui peut être conformé pour constituer un manchon de préhension. Ce corps 1 peut être rectiligne (figure 1) ou coudé (figure 2).

A l'intérieur du logement la ménagé dans ce corps, est logée une fibre optique 2 entourée d'un support tubulaire ou gaine 3.

De manière avantageuse et préférée, un conduit 4 est également installé ou ménagé à l'intérieur du corps 1, parallèlement ou sensiblement parallèlement à la fibre optique 2.

La pièce à main comprend également une tête de travail 5 qui peut être rapportée,par exemple par vissage, sur l'une des extrémités du corps 1, ou qui peut être formée par une conformation (figure 2) ou par un agencement (figure 1) de ladite extrémité.

A l'intérieur de cette tête de travail, est installé un dispositif optique de focalisation 6 qui, selon la figure 1 est constitué par un miroir convergent porté par un support 7 fixé par vissage sur la tête 5 et qui, selon la figure 2 est constitué par une lentille convergente vissée dans ladite tête.

La tête 5 est agencée pour permettre le positionnement d'un instrument optique d'application interchangeable 8 constitué par une fibre optique souple. La tête 5 est, par exemple, pourvue d'un taraudage 5a permettant la fixation, par vissage, de la fibre optique souple 8 dont l'extrémité de fixation 8a peut être filetée ou équipée d'un support fileté 9.

Les fibres optiques souples 8 sont appelées à remplir une fonction comparable à celle des limes des contre-angles mécaniques actuellement utilisées en odontologie. Elles peuvent avoir une forme cylindrique ou une forme conique rappelant celle desdites limes. Lorsqu'elles se trouvent positionnées sur la tête 5, elles s'étendent hors de ladite tête, sur la plus grande partie de leur longueur, de façon à pouvoir pénétrer à l'intérieur des canaux dentaires, en s'adaptant à la conformation particulière de ces derniers.

De manière avantageuse, ces fibres ont un diamètre de l'ordre de 20 à 60 centièmes de millimètre et présentent une conicité de l'ordre de 32 centièmes de millimètre sur une distance de 16 millimètres.

L'extrémité de la fibre optique 2 d'acheminement du rayon laser aboutit au dispositif de focalisation 6.

Selon le mode d'exécution illustré à la figure 1, l'extrémité de la fibre optique 2 est orientée de manière à diriger le rayon laser R sur le miroir convergent 6 lequel permet sa transformation en un faisceau convergent R1 et la réflexion de ce faisceau convergent en direction de l'extrémité interne de la fibre optique d'application 8 laquelle permet de guider ledit rayon focalisé jusqu'au voisinage de son extrémité libre où se trouve le point focal 0.

Selon le mode d'exécution représenté à la figure 2, l'extrémité de la fibre optique 2 est orientée de façon à diriger le rayon laser sur la lentille convergente 6 laquelle permet sa transformation en un faisceau convergent R1 dirigé sur l'extrémité interne de la fibre optique d'application 8.

On comprend que la fibre optique souple 8 permet d'amener le rayonnement laser à l'intérieur des canaux dentaires, y compris jusqu'aux extrémités des racines coudées.

La longueur d'onde 0,532 µm permettra de couper les tissus pulpaires et de coaguler les hémorragies.

La longueur d'onde 1,060 µm permettra d'élargir les parois canalaires.

La longueur d'onde 0,532 µm en milieu acqueux, permettra d'obtenir une cavitation pour nettoyer les parois.

Le conduit 4 destiné à amener le fluide de refroidissement tel que mélange air/eau jusqu'à la tête de travail 5, comporte une extrémité coudée 4a orientée en direction de la partie inférieure de la fibre optique souple 8, de façon à permettre la projection de ce mélange sur l'emplacement d'intervention.

La source d'émission comprend, par exemple, une source de lumière monochromatique 21 telle qu'un laser YAG Nd produisant un rayonnement sur une longueur d'onde de 1,06 µm, et raccordée à une source d'énergie 22 de commande fournissant des impulsions suffisantes pour que la source 21 produise des impulsions lumineuses conformes aux paramètres désirés.

Selon une autre disposition caractéristique de l'invention, une platine 23 capable d'influer sur le rayonnement laser au point de doubler sa fréquence, coulisse sur la source 21 et est solidaire d'une poignée de manoeuvre 24 qui permet de la faire coulisser entre la position représentée sur la figure 1 où la platine 23 est intercalée dans le trajet de la lumière entre la source 21 et la fibre 2, et une position de retrait où la platine 23 ne coupe plus ladite trajectoire. Grâce à cette disposition simple, la pièce à main dispose de la faculté d'appliquer un rayonnement de 1,06 µm ou de 0,532 µm sur le site d'intervention, de telle sorte qu'il suffit d'une seule source laser pour réaliser les procédures de manière sélective avec l'une ou l'autre des longueurs d'onde de rayonnement précitées.

De l'exposé qui précède, il ressort que l'appareillage ou installation selon l'invention, est plus généralement remarquable par le fait que cet appareillage comprend un moyen de production d'un rayonnement laser sur une longueur d'ondes permettant une plus forte absorption par l'hydroxyapatite que par l'eau ; un moyen associé audit moyen de production tel que ce dernier produise une succession d'impulsions du rayonnement produit, qui présentent une quantité d'énergie, une durée d'impulsion et une fréquence d'impulsions choisies de manière à couper le tissu dentaire sans entrainer d'effets secondaires nuisibles ;
et un moyen permettant de concentrer le rayonnement sur le tissu en un point suffisamment petit pour provoquer la découpe de ce dernier.

On a décrit ci-dessus une application très intéressante de l'invention à des traitements dentaires, mais on souligne que l'appareillage précédemment décrit peut également avantageusement être mis en oeuvre dans la préparation et la mise en place des prothèses dentaires, ainsi que pour la coupe ou la vaporisation de matériaux et tissus divers nécessitant une grande précision.

## Revendications

1. Installation utilisant le rayonnement laser pour la coupe des tissus dentaires, en particulier de l'émail et de la dentine, comprenant :
- un moyen (22) pour la production d'une succession d'impulsions du rayonnement produit avec un niveau d'énergie, une durée d'impulsion et une fréquence d'impulsions choisis pour couper le tissu dentaire ;
- un moyen (6) pour concentrer les impulsions du rayonnement sur le tissu dentaire en un point suffisamment petit pour provoquer la coupe de ce dernier ; caractérisée en ce qu'elle comprend :
- un moyen (21) pour la production d'un rayonnement laser à une longueur d'onde qui est absorbée plus fortement par l'hydroxyapatite que par l'eau ; et
- des moyens (20, 4, 4a) pour projeter un mélange d'air et d'eau dans la région du point durant au moins une partie du temps pendant lequel le rayonnement est concentré sur le tissu dentaire, de sorte que les impulsions de ce rayonnement puissent couper le tissu dentaire, sans causer d'effets secondaires nuisibles.

2. Installation selon la revendication 1, caractérisée en ce qu'elle comprend un laser spécifique de type YAG présentant, en combinaison, les caractéristiques ci-après :
- une longueur d'onde de l'ordre de 1,060 µm ou de l'ordre de 0,532 µm ;
- une capacité d'émission d'une énergie pulsée comprise entre 0 et 250 millijoules et, de préférence, entre 10 et 50 mJ ;
- une capacité de cadence ou fréquence de tir de l'ordre de 50 Hz ;
- des durées des pulsations ou impulsions comprises entre 100 et 300 micro-secondes.

3. Installation selon l'une des revendications 1 ou 2, comportant également une pièce à main comprenant un corps creux (1) de forme allongée, dont l'une des extrémités constitue ou est agencée pour constituer une tête (5) dans laquelle est installé un dispositif optique de focalisation (6) auquel aboutit l'extrémité de la fibre optique (2) logée dans ledit corps (1) et permettant de transporter le rayon laser jusqu'audit dispositif de focalisation (6), caractérisée en ce que cette tête (5) est agencée pour recevoir un instrument d'application interchangeable complétant la pièce à main et constitué par une fibre optique souple (8) dont une partie s'étend hors de ladite tête ; le dispositif de focalisation (6) étant orienté de manière à diriger le rayon laser focalisé sur l'extrémité interne de ladite fibre optique interchangeable, lorsque cette dernière est en place.

4. Installation selon la revendication 3, caractérisée en ce que les fibres optiques souples d'application (8) ont une forme conique, afin de remplir une fonction à l'intérieur des canaux dentaires.

5. Installation selon l'une quelconque des revendications 1 à 4, caractérisée en ce qu'elle comprend des moyens optiques (23, 24) permettant de modifier la longueur d'onde de son rayonnement laser, de manière à pouvoir passer d'une longueur d'onde de 1,060 µm à une longueur d'onde de 0,532 µm, et vice-versa, à partir d'une unique source de rayonnement.

6. Installation selon la revendication 3, caractérisée en ce que la pièce à main est pourvue d'un conduit (4) permettant d'amener le fluide de refroidissement jusqu'à la tête de travail (5) de ladite pièce à main, ce conduit comportant une extrémité coudée (4a) orientée en direction de la partie inférieure de la fibre souple d'application (8) positionnée sur ladite tête de travail.

## Claims

1. Installation employing laser radiation to cut dental tissues, in particular enamel and dentine, comprising :
- a means (22) for producing a series of radiation pulses produced with an energy level, a pulse duration and a pulse frequency selected to cut the dental tissue ;
- a means (6) for concentrating the radiation pulses onto the dental tissue in a point sufficiently small to cause the latter to be cut ; characterised by comprising :
- a means (21) for producing laser radiation at a wavelength which is absorbed to a greater degree by hydroxyapatite than by water ; and
- means (20, 4, 4a) for spraying a mixture of air and water into the region of the point for at least part of the time that the radiation is concentrated on the dental tissue, thereby enabling said pulses of radiation to cut the dental tissue without producing harmful side-effects.

2. Installation according to claim 1, characterised in that it comprises a special YAG-type laser exhibiting, in combination, the following features :
- a wavelength of the order of 1.060 µm or of the order of 0.532 µm ;
- an ability to emit a pulsed energy of between 0 and 250 millijoules and, preferably, between 10 and 50 mJ ;
- an ability to fire at a rate or frequency of the order of 50 Hz ;
- pulse or impulse lengths between 100 and 300 micro-seconds.

3. Installation according to either of claims 1 and 2, likewise including a handpiece comprising a hollow body (1) of elongated shape, one of the ends of which constitutes, or is arranged so as to constitute, a head (5) in which is fitted an optical focussing device (6) at which the end of the optical fibre (2) housed in said body (1) terminates and which allows the laser beam to be transported as far as said focussing device (6), characterised in that said head (5) is arranged so as to receive an interchangeable application instrument which complements the handpiece and constituted by a flexible optical fibre (8), part of which extends outside said head ; the focussing device (6) is aligned so as to direct the focussed laser beam onto the inner end of said interchangeable optical fibre once the latter is in position.

4. Installation according to claim 3, characterised in that the flexible optical application fibres (8) have a conical shape, in order to carry out a task inside the dental canals.

5. Installation according to any of claims 1 to 4, characterised in that it comprises optical means (23, 24) allowing the wavelength of its laser radiation to be modified, thus enabling a wavelength of 1.060 µm to be replaced by a wavelength of 0.532 µm, and vice versa, from a single radiation source.

6. Installation according to claim 3, characterised in that the handpiece is provided with a conduit (4) allowing the cooling fluid to be conveyed as far as the operating head (5) of said handpiece, said conduit having an elbowed end (4a) orientated towards the lower part of the flexible application fibre (8) positioned on said operating head.

## Patentansprüche

1. Vorrichtung zum schneiden von Zahngeweben, insbesondere Zahnschmelz und Zahnbein unter Verwendung von Laserstrahlen; umfassend:
- ein Mittel (22) zur Erzeugung einer Reihe von Strahlenimpulsen, die mit einem Energieniveau, einer Impulsdauer und einer Impulsfrequenz erzeugt werden, die geeignet zum Schneiden von Zahngewebe gewählt sind;
- ein Mittel (6), um die Strahlenimpulse auf einen Punkt des Zahngewebes zu konzentrieren, der ausreichend klein ist, um dessen Zerschneiden zu bewirken; dadurch gekennzeichnet, daß die Vorrichtung
- ein Mittel (21) zur Erzeugung von Laserstrahlen mit einer Wellenlänge umfaßt, die stärker von Hydroxyapatit als von Wasser absorbiert wird; und
- Mittel (20, 4, 4a), um zumindest während eines Teils der Zeit, in der die Strahlen auf das Zahngewebe konzentriert sind, eine Mischung aus Luft und Wasser in den Bereich des Schneidepunktes zu sprühen, so daß diese Strahlenimpulse das Zahngewebe schneiden können, ohne schädliche Nebenwirkungen zu verursachen.

2. Vorrichtung nach Anspruch 1, dadurch gekennzeichnet, daß sie einen speziellen Laser vom Typ YAG umfaßt und in Kombination damit folgende Merkmale aufweist:
- eine Wellenlänge der Größenordnung von 1,060 µm oder der Größenordnung von 0,532 µm;
- eine Emissionsleistung einer gepulsten Energie zwischen 0 und 250 Milli-Joules und bevorzugt zwischen 10 und 50 mJ;
- eine Taktleistung oder Schußfrequenz in der Größenordnung von 50 Hz;
- Impulslängen oder Impulse zwischen 100 und 300 Mikrosekunden.

3. Vorrichtung nach einem der Ansprüche 1 oder 2, die des weiteren ein Handstück mit einem länglich geformten Hohlkörper (1) umfaßt, wobei eines seiner Enden einen Kopf (5) bildet oder so gestaltet ist, daß es einen Kopf (5) bildet, in den eine optische Fokussierungseinrichtung (6) eingebaut ist, in die das Ende einer Lichtleitfaser (2) einmündet, die im Körper (1) positioniert ist und die Weiterleitung des Laserstrahls bis zur Fokussierungseinrichtung (6) ermöglicht, dadurch gekennzeichnet, daß dieser Kopf (5) so ausgebildet ist, daß er ein das Handstück vervollständigendes auswechselbares Anwendungsinstrument aufnimmt, das aus einer flexiblen Lichtleitfaser (8) gebildet wird, wobei eines ihrer Enden aus dem Kopf herausragt; wobei die Fokussierungseinrichtung (6) so ausgerichtet ist, daß der fokussierte Laserstrahl auf das innere Ende der auswechselbaren Lichtleitfaser gelenkt wird, wenn diese eingesetzt ist.

4. Vorrichtung nach Anspruch 3, dadurch gekennzeichnet, daß die flexiblen Anwendungs-Lichtleitfasern (8) eine konische Form aufweisen, um im Inneren der Zahnkanäle eine Funktion auszuüben.

5. Vorrichtung nach einem der Ansprüche 1 bis 4, dadurch gekennzeichnet, daß sie optische Mittel (23, 24) umfaßt, die eine Modifizierung der Wellenlänge der Strahlen ermöglichen, so daß von einer einzigen Strahlenquelle aus von einer Wellenlänge von 1,060 µm zu einer Wellenlänge von 0,532 µm und umgekehrt übergegangen werden kann.

6. Vorrichtung nach Anspruch 3, dadurch gekennzeichnet, daß das Handstück mit einer Leitung (4) versehen ist, welche die Zufuhr der Kühlflüssigkeit zum Arbeitskopf (5) des Handstücks ermöglicht, wobei diese Leitung ein gekrümmtes Ende (4a) aufweist, das auf den inneren Teil der flexiblen, im Arbeitskopf positionierten Anwendungs-Lichtleitfaser (8) hin ausgerichtet ist.
